(19) Europäisches Patentamt
European Patent Office
Office européen des brevets

(11) **EP 4 102 729 A1**

(12) **DEMANDE DE BREVET EUROPEEN**

(43) Date de publication:
**14.12.2022 Bulletin 2022/50**

(51) Classification Internationale des Brevets (IPC):
***H04B 5/00*** *(2006.01)*

(21) Numéro de dépôt: **22177556.2**

(22) Date de dépôt: **07.06.2022**

(52) Classification Coopérative des Brevets (CPC):
**H04B 5/0031; H04B 5/0081**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité: **07.06.2021 FR 2105960**

(71) Demandeur: **Commissariat à l'énergie atomique et aux énergies alternatives**
**75015 Paris (FR)**

(72) Inventeurs:
• **CLEMENTE, Antonio**
**38054 GRENOBLE CEDEX 9 (FR)**
• **SMIERZCHALSKI, Maciej**
**38054 GRENOBLE CEDEX 9 (FR)**

(74) Mandataire: **Lavoix**
**2, place d'Estienne d'Orves**
**75441 Paris Cedex 09 (FR)**

(54) **DISPOSITIF ANTENNAIRE D'ILLUMINATION EN CHAMP PROCHE DE LA PEAU PAR ONDES MILLIMÉTRIQUES**

(57) L'invention concerne un dispositif antennaire (10) configuré pour superposer une pluralité de modes électromagnétiques en champ proche, avec une structure multicouche comprenant, selon une direction verticale d'empilement :
- une première couche (12) d'alimentation comprenant un premier diélectrique sur un circuit intégré pour application spécifique et sur une partie inférieure d'un circuit d'alimentation en courant continu,
- une deuxième couche d'alimentation comprenant une partie supérieure d'un circuit d'alimentation en courant continu,
- une troisième couche (16) comprenant un deuxième diélectrique sur un plan métallique (15),
- une quatrième couche (18) comprenant au moins un élément rayonnant planaire (19$_A$), le plan métallique (15) jouant le rôle de plan de masse,
- une cinquième couche (20) comprenant un radôme,
le dispositif antennaire comprenant au moins une ligne d'alimentation (17) s'étendant de la première à la quatrième couche.

FIG.1

EP 4 102 729 A1

**EP 4 102 729 A1**

**Description**

La présente invention concerne un dispositif antennaire d'illumination en champ proche de la peau par ondes millimétriques.

L'invention se situe dans le domaine des systèmes d'antennes focalisées en champ proche utiles dans un grand nombre d'applications, notamment pour les radars dédiés à des applications médicales et les dispositifs médicaux basés sur les ondes électromagnétiques et les radiofréquences. De tels dispositifs médicaux sont propres à être utilisés pour le diagnostic (détection du cancer, attaque cérébrale, etc.) ou également pour le traitement et la thérapie (stimulation des endorphines, thérapie du cancer, thérapie des fractures osseuses, etc.)

Les techniques basées sur les micro-ondes sont en général non invasives, mais nécessitent des solutions d'antenne innovantes pour faire face aux pertes de pénétration élevées dues à la présence de la peau et d'autres milieux inhomogènes. Plus généralement, dans le cas des systèmes radar pour application médicale, les technologies d'antennes focalisées en champ proche sont propres à être utilisées pour augmenter les capacités de transfert et/ou de focalisation de l'énergie électromagnétique dans les milieux inhomogènes (tissus humains ou à la surface de la peau, milieux stratifiés, etc.)

Dans l'état de la technique, on connaît des antennes focalisées en champ proche, notamment pour application dans les futurs systèmes de communication associés aux réseaux de communication de sixième génération qui requièrent également des surfaces intelligentes reconfigurables propres à fonctionner en champ proche pour régler et gérer au mieux les caractéristiques des ondes électromagnétiques impliquées et contrôler dynamiquement le canal de propagation.

Pour les applications médicales, on connaît des antennes focalisées en champ proche telles qu'une simple antenne cornet comme source de champ proche, ou encore des dispositifs mobiles plus récents pour thérapie par ondes millimétriques dont la fréquence est inférieure à 300 GHz, utilisant une antenne réseau tel que le dispositif décrit dans le document WO 3071 163 A1 où le réseau utilise un réseau d'alimentation de jonctions en T qui mélange les couches de signaux radiofréquence RF et en courant continu (DC de l'anglais *direct current*). Cependant, la solution selon le document WO 3071 163 A1 présente des pertes et nécessite notamment quatre circuit intégrés spécialisés ASIC (de l'anglais *application-specific integrated circuit*) de 20 mW.

L'invention a pour objet de remédier aux inconvénients de l'état de la technique en proposant une architecture alternative d'antenne en champ proche pour l'illumination uniforme et ponctuel de la peau, propre à réduire les pertes de même que le nombre d'ASIC(s) nécessaire(s) à son alimentation et le coût associé, tout en restant compact. Autrement dit, l'invention a pour objectif de proposer une architecture alternative d'antenne propre à générer une distribution de champ électromagnétique spécifique, en amplitude ou en amplitude et en phase, sur ou dans un milieu stratifié et inhomogène (i.e. dans un plan spécifique situé à une distance définie de l'ouverture rayonnante et sur une couche spécifique du milieu stratifié).

A cet effet, l'invention propose un dispositif antennaire d'illumination en champ proche de la peau par ondes millimétriques dans lequel ledit dispositif est configuré pour superposer une pluralité de modes électromagnétiques en champ proche, en utilisant une structure multicouche comprenant, selon une direction verticale d'empilement :

- une première couche d'alimentation comprenant au moins un premier diélectrique sur un circuit intégré pour application spécifique et sur une première partie inférieure d'un circuit métallique d'alimentation en courant continu,
- une deuxième couche d'alimentation en courant continu, superposée sur la première couche d'alimentation selon la direction verticale d'empilement, et comprenant une deuxième partie supérieure d'un circuit métallique d'alimentation en courant continu,
- une troisième couche, superposée sur la deuxième couche d'alimentation selon la direction verticale d'empilement, et comprenant un deuxième diélectrique sur un plan métallique,
- une quatrième couche comprenant au moins un élément rayonnant planaire dudit dispositif antennaire, la quatrième couche étant superposée sur la troisième couche selon la direction verticale d'empilement, le plan métallique de la troisième couche jouant le rôle de plan de masse de l'élément rayonnant,
- une cinquième couche, superposée sur la quatrième couche selon la direction verticale d'empilement, et comprenant un radôme propre à séparer ledit au moins un élément rayonnant de la peau,

le dispositif antennaire comprenant en outre au moins une ligne d'alimentation s'étendant verticalement de la première couche à la quatrième couche.

Avantageusement, l'architecture (i.e. l'empilement) du dispositif antennaire d'illumination en champ proche de la peau par ondes millimétriques proposé est ainsi, une fois alimentée et activée, configurée pour superposer une pluralité de modes électromagnétiques et pour minimiser les pertes via le plan métallique de la troisième couche qui joue également le rôle de plan de masse de l'élément rayonnant.

Autrement dit, selon la présente invention, le dispositif antennaire proposé combine dans la région de champ proche une combinaison linéaire d'une série de modes rayonnants pour satisfaire les exigences particulières de distribution du rayonnement, chaque mode étant excité d'une manière spécifique en amplitude et en phase, au moyen de ladite architecture, pour construire correctement la distribution de champ requise.

Le dispositif antennaire d'illumination en champ proche de la peau par ondes millimétriques peut également présenter une ou plusieurs des caractéristiques ci-dessous, prises indépendamment ou selon toutes les combinaisons techniquement envisageables :

- les modes électromagnétiques propres à être superposés sont des modes cylindriques ;
- ledit au moins un élément rayonnant planaire est un élément rayonnant à ondes fuyantes correspondant à au moins un patch à fente(s) annulaire(s), ou à au moins un patch à tronçons transversaux continus, ledit au moins un « patch à tronçons transversaux continus » étant connu sous l'acronyme CTS (de l'anglais *continuous transverse stub*) ou encore selon l'une des appellations « réseau de stubs continus transverses », ou « réseaux à stub transversal continu » ;
- la troisième couche comprend au moins une rangée de via(s) métallique(s) transversale définie par la direction verticale d'empilement et une direction transversale perpendiculaire à la direction verticale d'empilement, ladite au moins une rangée de via(s) métallique(s) transversale s'étendant verticalement du plan de masse métallique de la troisième couche au plan de la quatrième couche comprenant ledit au moins un élément rayonnant planaire ;
- ledit au moins un élément rayonnant planaire est un élément rayonnant à ondes fuyantes correspondant à au moins un patch à fente(s) annulaire(s), la troisième couche comprenant, de part et d'autre dudit au moins un patch à fente(s) annulaire(s), au moins une rangée de via(s) métallique(s) transversale s'étendant verticalement du plan de masse métallique au plan de la quatrième couche comprenant ledit au moins un patch à fente(s) annulaire(s), ladite au moins une ligne d'alimentation s'étendant verticalement du centre de la première couche d'alimentation au centre dudit au moins un patch à fente(s) annulaire(s);
- ledit au moins un élément rayonnant planaire est un élément rayonnant à ondes fuyantes correspondant à au moins un patch à tronçons transversaux continus, la troisième couche comprenant, de part et d'autre dudit au moins un patch à tronçons transversaux continus, au moins une rangée de via(s) métallique(s) transversale s'étendant verticalement du plan de masse métallique au plan de la quatrième couche comprenant ledit au moins un patch à tronçons transversaux continus, ladite au moins une ligne d'alimentation s'étendant verticalement du centre de la première couche au centre de la quatrième couche comprenant ledit au moins un patch à tronçons transversaux continus, les fentes dudit au moins un patch à tronçons transversaux continus étant réparties symétriquement de part et d'autre du point d'alimentation fourni par la ligne d'alimentation au sein de la quatrième couche ;
- ledit au moins un élément rayonnant planaire est un élément rayonnant à ondes fuyantes correspondant à au moins un patch à tronçons transversaux continus, la troisième couche comprenant, localisée sensiblement à proximité d'une première extrémité transversale de ladite troisième couche, au moins une rangée de via(s) métallique(s) transversale s'étendant verticalement du plan de masse métallique au plan de la quatrième couche comprenant ledit au moins un patch à tronçons transversaux continus, ladite au moins une ligne d'alimentation, s'étendant verticalement de la première couche à la quatrième couche en étant sensiblement localisée au niveau d'une deuxième extrémité transversale, de la troisième couche, parallèle et opposée à la première extrémité transversale de ladite troisième couche localisée sensiblement à proximité de ladite au moins une rangée de via(s) métallique(s) transversale, ladite au moins une ligne d'alimentation étant propre à exciter le premier mode transverse magnétique dudit au moins un patch à tronçons transversaux continus ;
- le dispositif comprend en outre entre ladite au moins une rangée de via(s) métallique(s) transversale et ladite au moins une ligne d'alimentation au moins deux autres vias métalliques s'étendant verticalement du plan de masse métallique au plan de la quatrième couche comprenant ledit au moins un patch à tronçons.
- ledit au moins un élément rayonnant planaire est un élément rayonnant à ondes fuyantes correspondant à au moins un patch à tronçons transversaux continus, la troisième couche comprenant, localisé sensiblement à proximité d'une première extrémité transversale de ladite troisième couche, au moins une rangée de via(s) métallique(s) transversale s'étendant verticalement du plan de masse métallique au plan de la quatrième couche comprenant ledit au moins un patch à tronçons transversaux continus, et comprenant au moins deux guides d'onde longitudinaux obtenus par insertion au sein de la troisième couche d'au moins une autre rangée de via(s) métallique(s) longitudinale perpendiculaire, selon une direction longitudinale, à la fois à ladite au moins une rangée de via(s) métallique(s) transversale et au plan de masse métallique, ladite au moins une autre rangée de via(s) métallique(s) longitudinale s'étendant verticalement du plan de masse métallique au plan de la quatrième couche comprenant ledit au moins un patch à tronçons transversaux continus ;

- ladite au moins une rangée de via(s)métallique(s) longitudinale s'étend longitudinalement à partir de ladite au moins une rangée de via(s) métallique(s) transversale et au-delà de la dernière fente dudit au moins un patch à tronçons transversaux continus tout en s'arrêtant à distance d'une deuxième extrémité transversale, de la troisième couche, parallèle et opposée à la première extrémité transversale de ladite troisième couche à localisée sensiblement à proximité de ladite au moins une rangée de via(s) métallique(s) transversale, ladite au moins une ligne d'alimentation, s'étendant verticalement de la première couche à la quatrième couche en étant localisée longitudinalement entre :

- l'extrémité de ladite au moins une rangée de via(s) métallique(s) longitudinale opposée à ladite au moins une rangée de via(s) métallique(s) transversale, et
- la deuxième extrémité transversale, de la troisième couche, parallèle et opposée à la première extrémité transversale de ladite troisième couche localisée sensiblement à proximité de ladite au moins une rangée de via(s) métallique(s) transversale ;

- ladite au moins une rangée de via(s)métallique(s) longitudinale s'étend longitudinalement à partir de ladite au moins une rangée de via(s) métallique(s) transversale et au-delà de la deuxième extrémité transversale, de la troisième couche, parallèle et opposée à la première extrémité transversale de ladite troisième couche localisée sensiblement à proximité de ladite au moins une rangée de via(s)métallique(s) transversale, le dispositif antennaire comprenant alors au moins deux lignes d'alimentation, s'étendant verticalement de la première couche à la quatrième couche en étant localisées transversalement de part et d'autre de ladite au moins une rangée de via(s)métallique(s) longitudinale.
- les modes électromagnétiques propres à être superposés comprennent en outre des modes cavité propres à être excités par ajout d'au moins une cavité métallique au sein de la structure multicouche ;
- le radôme est configuré pour être une couverture diélectrique modulée.

D'autres caractéristiques et avantages de l'invention ressortiront de la description qui en est donnée ci-dessous, à titre indicatif et nullement limitatif, en référence aux figures annexées, parmi lesquelles :

[Fig 1] la figure 1 représente schématiquement l'architecture du dispositif antennaire d'illumination en champ proche de la peau par ondes millimétriques selon un premier mode de réalisation ;
[Fig 2] [Fig 3] les figures 2 et 3 illustrent en perspective et en coupe une telle architecture dans lequel l'élément rayonnant planaire est un élément rayonnant à ondes fuyantes correspondant à un patch à fente(s) annulaires ;
[Fig 4] [Fig 5] les figures 4 et 5 illustrent en perspective différentes variantes de l'architecture proposée dans lequel l'élément rayonnant planaire est un élément rayonnant à au moins un patch à tronçons transversaux continus ;
[Fig 6] [Fig 7] les figures 6 et 7 illustrent différentes variantes d'un mode de réalisation de l'architecture proposée dans lequel les modes électromagnétiques propres à être superposés selon l'invention comprennent en outre des modes cavité propres à être excités par ajout d'au moins une cavité métallique au sein de la structure multicouche selon la présente invention ;
[Fig 8] la figure 8 illustre une variante de réalisation selon laquelle le radôme est configuré pour être une couverture diélectrique modulée.

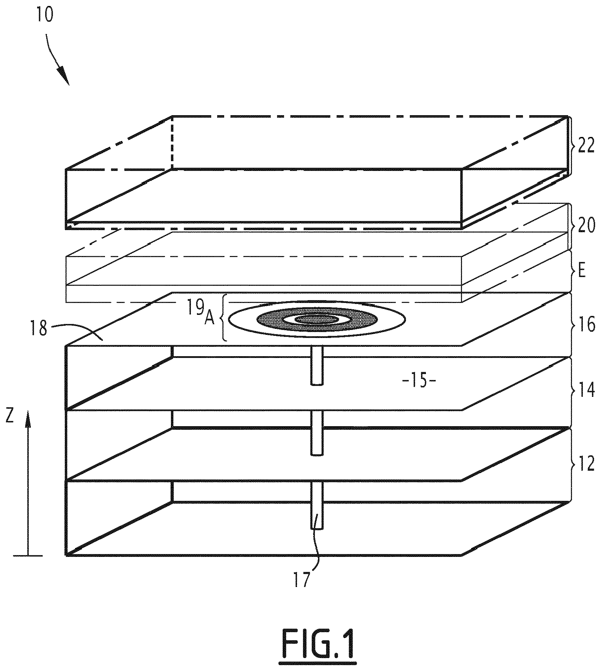

La figure 1 représente schématiquement l'architecture générale du dispositif 10 antennaire d'illumination en champ proche de la peau de thérapie par ondes millimétriques selon la présente invention.

La configuration générale d'un tel dispositif 10 est basée sur une structure multicouche compacte comprenant notamment au moins un élément radiofréquence RF, un système d'alimentation, une source d'antenne, un radôme (i.e. notamment un couvercle en plastique) séparant le dispositif antennaire 10 de la peau et un modèle de peau, le dispositif 10 fonctionnant dans la zone réactive de l'antenne.

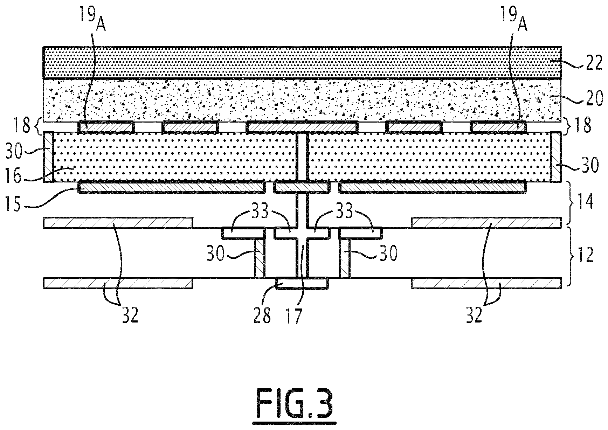

Plus précisément, selon la présente invention, la structure multicouche compacte du dispositif antennaire 10 comprend, selon une direction verticale Z d'empilement comprend tout d'abord une première couche 12 d'alimentation comprenant un premier diélectrique sur un circuit intégré pour application spécifique ASIC (de l'anglais *application-specific integrated circuit*) et une première partie inférieure du circuit métallique d'alimentation en courant continu. Autrement dit, la première couche 12 comprend un premier substrat diélectrique reposant en face inférieure sur une métallisation comprenant l'ASIC ainsi que sur une première partie inférieure du circuit métallique d'alimentation en courant continu, tel qu'illustré par la suite par la figure 3.

La puissance de sortie radiofréquence de l'ASIC de la première couche 12 est notamment de 20 mW propre à fournir un flux de puissance minimum de 50 W/m2 pour obtenir une interaction avec la peau par illumination de plus de 95% de sa surface environ.

De plus, la structure multicouche compacte du dispositif antennaire 10 comprend en outre une deuxième couche métallique d'alimentation en courant continu, superposée sur le substrat de la première couche 12 d'alimentation selon la direction verticale Z d'empilement, et comprenant, de sorte que la face supérieure du substrat de la couche 12 comprend une deuxième partie supérieure du circuit métallique d'alimentation en courant continu.

De plus, la structure multicouche compacte du dispositif antennaire 10 de la figure 1 comprend en outre une troisième couche 16, superposée sur la deuxième couche métallique d'alimentation selon la direction verticale d'empilement Z, et comprenant un deuxième diélectrique sur un plan métallique 15.

Par ailleurs, le dispositif antennaire 10 comprend en outre au moins une ligne métallique d'alimentation directe 17 s'étendant verticalement de la première couche 12 à une quatrième couche 18 comprenant au moins un élément métallique rayonnant planaire $19_A$ dudit dispositif antennaire 10, la quatrième couche 18 étant superposée sur la troisième couche 16 selon la direction verticale d'empilement z, le plan métallique 15 de la troisième couche 16 jouant le rôle de plan de masse de l'élément rayonnant $19_A$.

Plus précisément, selon une variante particulière, sur la deuxième couche métallique comprenant la partie supérieure de l'alimentation en courant continu est superposée également un film préimprégné 14 (de l'anglais *prepreg*) permettant de solidariser par collage le substrat de la première couche 12 à celui de la troisième couche 16 afin d'optimiser l'utilisation de leur plans métalliques respectifs. Autrement dit, l'empilement des couches 12, 14, 16 et 18 selon la présente invention comprend selon cette variante deux substrats diélectriques des couches 12 et 16 et cinq niveaux distincts de métallisation correspondant respectivement à l'ASIC, aux deux niveaux inférieurs et supérieurs de circuit métallique d'alimentation en courant continu localisés verticalement de part et d'autre du substrat de la couche 12, au plan métallique 15, et audit au moins un élément rayonnant $19_A$.

Selon une autre variante, la couche 14 correspond à une couche supplémentaire de substrat permettant l'utilisation de techniques de collage alternative au préimprégné tel qu'un collage de type haute température, ou un frittage de céramiques à basse température (de l'anglais *Low temperature co-fired ceramic*).

Autrement dit, l'empilement des couches 12, 14 et 16 au travers duquel s'étend verticalement ladite au moins une ligne d'alimentation 17 est dédié à l'alimentation de la couche 18 radiofréquence RF, et responsable de la distribution de la puissance audit au moins un élément rayonnant $19_A$. Une telle alimentation correspond par exemple à une alimentation en série ou en parallèle mise en œuvre selon différentes techniques de réalisation comme les jonctions en T des lignes ruban ou les ondes de propagation associées à des guides d'ondes intégré à chaque substrat SIW (de l'anglais *Substrate Integrated Waveguide)*.

Selon un aspect particulier, en présence d'un film de liaison préimprégné au sein de la couche 14, propre à produire une discontinuité de la masse, pour optimiser la transition entre l'ASIC de la première couche 12 et la ligne d'alimentation 17, dont la base est équivalente à un coaxial, et assurer une continuité de la masse, des plots de couplage (de l'anglais *coupling stub*) non représentés sur la figure 1, et illustrés par la suite sur la figure 3 (avec les vias métalliques 30 de la couche 12), sont propres à être insérés autour de la ligne d'alimentation 17 pour implémenter cette transition.

La quatrième couche 18 de rayonnement est responsable de l'excitation des champs dans la zone réactive E correspondant à un espace libre au-dessus de la couche 18 comprenant au moins un élément rayonnant planaire. Comme détaillé par la suite, l'élément rayonnant de la quatrième couche 18 correspond, par exemple, à un réseau de patchs, et en particulier utilisant une ou plusieurs fente(s) pour générer un mode d'ordre donné dans la zone réactive, voire un ou plusieurs modes cavités.

De plus, la structure multicouche compacte du dispositif antennaire 10 de la figure 1 comprend en outre une cinquième couche 20, superposée sur la quatrième couche 18 de rayonnement et dont elle est séparée verticalement par un espace E, selon la direction verticale d'empilement Z, et comprenant un radôme propre à séparer ledit au moins un élément rayonnant de la peau illustrée par la couche 22 de la figure 1.

A titre d'alternative, l'espace E est propre à être remplacé par un film de collage.

Entre la peau 22 et la couche de rayonnement 18, une telle couche de couverture 20, notamment en plastique et par exemple de 2 mm d'épaisseur, est utilisée selon la présente invention pour isoler le corps humain du système de rayonnement correspondant au dispositif antennaire 10. En effet, pour des raisons médicales, la peau 22 ne peut pas toucher directement les parties métalliques du dispositif antennaire 10. De plus, si la peau 22 est humide, la présence d'une telle couche de couverture 20 est propre à éviter de dégrader les performances du dispositif antennaire 10.

La cinquième couche 20, traitée comme un milieu d'adaptation semi-fini et délimitée par la peau et la couche de rayonnement 18, a un impact crucial sur la distribution de puissance sur la peau 22 dans la zone réactive.

Selon un aspect particulier optionnel, la cinquième couche 20 est également propre à présenter un changement d'index graduel.

Ainsi, les couches supérieures, 16, 18 et 20 de rayonnement et de couverture permettent de concevoir la partie radiative RF du dispositif antennaire 10, tandis que les couches métalliques inférieures de part et d'autre de la couche 12 sont utilisées pour le routage du courant continu et l'intégration de l'émetteur-récepteur RF en termes de circuit.

Par rapport au dispositif antennaire décrit dans le document WO 3071 163 A1, basé sur un système d'alimentation à jonction en T et une antenne patch, où il existe un fort couplage mutuel entre les couches RF et DC, le plan métallique 15, introduit selon la présente invention pour jouer à la fois le rôle de séparation entre la partie supérieure RF composée des couches 16, 18 et 20, et la partie inférieure composée des couches 12 d'alimentation et 14 de solidarisation, et pour jouer le rôle de plan de masse dudit au moins un élément rayonnant $19_A$ permet une réduction du couplage mutuel entre les couches RF et DC et une minimisation des pertes.

La couche de peau 22 de la figure 1, modélisant la peau, correspond à un modèle de peau connu associé à une permittivité équivalente, une conductivité et une tangente de perte à une fréquence donnée. De tels paramètres sont également propres à prendre en compte les différents taux de concentration d'humidité dans la peau.

Avantageusement, comme décrit plus en détail par la suite en relation avec la figure 3, le dispositif antennaire 10 présente une taille de 10 x 10 mm² et ne nécessite qu'un ASIC source de 20 mW alors que les dispositifs antennaire actuels d'illumination en champ proche de la peau par ondes millimétriques, notamment le dispositif antennaire décrit dans le document WO 3071 163 A1 nécessitent un nombre supérieur d'ASICs de 20 mW.

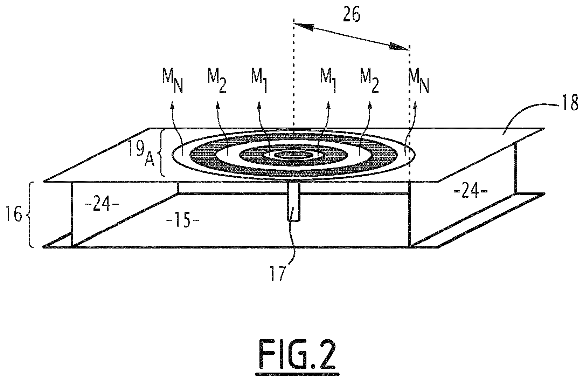

Selon un aspect particulier illustré par les figures 2 à 5, ledit au moins un élément rayonnant planaire de la quatrième couche 18 est un élément rayonnant à ondes fuyantes correspondant à au moins un patch à fente(s) annulaire(s) $19_A$ ou à au moins un patch à tronçons transversaux continus $19_B$ ou encore à un patch circulaire, un anneau ou tout autre élément rayonnant à ondes fuyantes.

Ledit au moins un « patch à tronçons transversaux continus » est connu sous l'acronyme CTS (de l'anglais *continuous transverse stub*) ou encore selon l'une des appellations « réseau de stubs continus transverses », ou « réseaux à stub transversal continu ».

Un élément rayonnant à ondes fuyantes présente l'avantage d'être compatible avec l'architecture de dispositif antennaire 10 proposée selon la présente invention basée, comme indiqué précédemment, sur une séparation entre les couches supérieures 16, 18 de rayonnement et 20 de couverture permettant de concevoir la partie radiative RF du dispositif antennaire 10, et les couches inférieures 12 et 14 utilisées pour le routage du courant continu et l'intégration de l'émetteur-récepteur RF en termes de circuit, et ce avec cinq niveaux distincts de métallisation correspondant respectivement à l'ASIC, aux deux circuits métallique inférieurs et supérieurs d'alimentation en courant continu dédiées tous deux à la division de puissance, au plan métallique 15, et audit au moins un élément rayonnant $19_A$ ou encore $19_B$.

Plus précisément, un élément rayonnant à ondes fuyantes est une structure monocouche en terme de substrat (i.e. la couche (i.e. le plan) 18 d'élément rayonnant reposant sur la troisième couche 16 de substrat diélectrique de la figure 1) ne nécessitant pas de diviseur de puissance complexe mais généralement une alimentation en série. La modulation appropriée ou l'utilisation de modes cylindriques d'ordre supérieur de ce type d'élément rayonnant permet avantageusement de distribuer efficacement le champ proche et de minimiser les pertes.

En particulier, le rayonnement d'un tel élément rayonnant à ondes fuyantes est obtenu par la transition d'une onde de surface (i.e. onde stationnaire) à une onde de fuite, ce qui permet de réduire les dimensions de l'élément rayonnant à ondes fuyantes. Le phénomène physique qui régit la distribution du champ proche est basé sur la superposition des modes dans la zone réactive/champ proche. En effet, les modes d'ordre supérieur lancés dans le milieu entre la peau et le dispositif antennaire 10 sont propres à se superposer de manière constructive lorsqu'ils sont excités avec une amplitude et une phase prédéterminée.

Selon un aspect particulier de la présente invention, les modes électromagnétiques propres à être superposés sont des modes cylindriques, les modes cylindriques ayant le modèle analytique le plus simple pour la sommation des modes.

En particulier, lorsque l'élément rayonnant à ondes fuyantes correspond à un patch circulaire, un anneau ou un patch à fentes annulaires $19_A$ tel qu'illustré par la figure 2, les modes cylindriques d'ordre supérieur propres à se superposer selon la présente invention dépendent du rayon de l'élément rayonnant, et pour un anneau le rayon et la taille de l'anneau définissent le numéro de mode rayonné.

Tel qu'illustré par la figure 2, l'élément rayonnant à ondes fuyantes, correspondant notamment à un patch à fente(s) annulaire(s) $19_A$, est excité en série par l'alimentation centrale 17 précitée qui fait circuler une onde dans le substrat diélectrique de la troisième couche 16 illustrée par la figure 1, de sorte à générer une distribution quasi-uniforme ou multi-spots du champ proche à l'intérieur de l'onde progressive alimentant le réseau concentrique de fentes annulaires de taille et de position définies du patch à fente(s) annulaire(s) $19_A$.

Les modes cylindriques sont classiquement définis par une source de potentiels électriques et/ou magnétiques vectoriels avec une variation périodique uniquement azimutale et la fonction d'onde cylindrique élémentaire est exprimée classiquement sous la forme suivante :

$$\gamma_{k_\rho,n} = H_n^{(2)} e^{-jk_\rho \rho} \begin{cases} \sin n\varphi \\ \cos n\varphi \end{cases} \rightarrow \frac{j^n e^{-jk_\rho \rho}}{\sqrt{\rho}} \begin{cases} \sin n\varphi \\ \cos n\varphi \end{cases}$$

où n est l'ordre du mode, les autres variables étant définies par M. Smierzchalski au sein du document intitulé « Characterization methods for metamaterials. Directive antennas using space eigenmodes » Univ. Rennes 1, 2014. Le champ rayonné par les modes cylindriques est proportionnel au potentiel vectoriel électrique (ou magnétique) A = $\hat{z}\gamma_{k_\rho,n}$ et en conséquence à la distribution $\cos n\varphi$. La réalisation de la variation en cosinus des champs est possible par une distribution circulaire du courant avec utilisation d'un anneau annulaire ou fendu tel qu'illustré par la figure 2.

La superposition des modes cylindriques pour le champ proche multi-spots et la distribution uniforme de la densité de puissance sur la peau mise en œuvre selon la présente invention suppose que chaque mode cylindrique est excité avec une amplitude $a_n$ et une phase $\varphi_n$ données.

Dans la synthèse du rayonnement de champ proche et de champ lointain, un potentiel vectoriel électrique total pour N modes cylindriques comme $A_{total} \approx \sum_{n=1}^{N} a_n \cos(n\varphi + \varphi_n)$ est notamment propre à être utilisé.

De plus, un ensemble approprié d'amplitude et de phase des modes cylindriques sélectionnées au préalable, permet de générer un faisceau crayon, un faisceau conique ou toute autre forme de motif de faisceau.

Comme illustré par la figure 2, chaque fente du patch à fente(s) annulaire(s) $19_A$ à N fentes dans le plan de masse infini 15, génère un mode cylindrique particulier $M_1$, $M_2$, $M_N$ respectivement déterminé par son rayon.

Une alimentation en série permet d'utiliser une seule excitation 17 qui alimente à la fois les N modes et permet d'éviter l'utilisation d'un réseau d'alimentation parallèle ou en série à N nœuds d'excitation complexe à mettre en œuvre et associé à des pertes.

Tel qu'illustré par la figure 2, selon un aspect particulier de la présente invention, la troisième couche 16 comprend au moins une rangée 24 de via(s) métallique(s) transversale définie par la direction verticale d'empilement z et une direction transversale perpendiculaire à la direction verticale d'empilement, ladite au moins une rangée de via(s) métallique(s) transversale s'étendant verticalement du plan de masse métallique 15 de la troisième couche 16 au plan de la quatrième couche 18 comprenant ledit au moins un élément rayonnant $19_A$.

Une telle rangée 24 de via(s) métallique(s) permet de réaliser un mur métallique, un tel mur métallique étant, à titre d'alternative, propre à être réalisé par une métallisation continue en lieu et place de ladite rangée 24 discontinue de via(s) métalliques.

En comparaison, un élément rayonnant à ondes fuyantes classique est ouvert aux extrémités transversales de la troisième couche 16 et l'onde progressive (ou onde de surface) est atténuée d'environ -20 dB avant d'atteindre le bord de la troisième couche 16, ce qui conduit le rayon (i.e. la dimension) de l'élément rayonnant à ondes fuyantes classique à plusieurs longueurs d'onde, et le rend encombrant. En particulier, une telle atténuation de 20 dB sur le bord nécessite classiquement un rayon d'antenne de plusieurs longueurs d'ondes, ce qui à 60 GHz peut correspondre à un rayon de l'ordre de 25 mm.

La présence de ladite au moins une rangée 24 de via(s) métallique(s) transversale proposée selon cet aspect particulier de la présente invention permet avantageusement de réduire considérablement la taille de l'antenne tout en créant, d'une part, une onde stationnaire à une fréquence donnée, conduisant à une solution à bande étroite, et en limitant d'autre part, le nombre de périodes de la surface modulée et les performances en champ lointain (faible gain et lobe secondaire élevé) et en champ proche (focalisation du faisceau).

Toutefois, pour l'application d'illumination en champ proche de la peau visée par la présente invention les effets associés à une telle réduction avantageuse de la taille n'ont pas d'impact sur l'application proposée. En effet, le dispositif antennaire 10 proposé selon la présente invention pour la thérapie millimétrique peut être à bande étroite puisqu'il fonctionne à 62,25 GHz dans une bande passante de 1,6 %. De plus, la petite taille de l'antenne est requise dans une telle application mobile où un faisceau de focalisation pour une pénétration profonde de la peau est nécessaire.

Ainsi, sur la figure 2, le réseau de fentes cylindriques de l'élément rayonnant à ondes fuyantes correspondant à un patch à fente(s) annulaire(s) $19_A$ est imprimé sur le substrat diélectrique de la troisième couche 16 mis à la terre via le plan de masse 15 et court-circuité sur chaque bord transversal par ladite au moins une rangée 24 de via(s) métallique(s). La broche coaxiale 17 alimente l'élément rayonnant à ondes fuyantes correspondant à un patch à fente(s) annulaire(s) $19_A$ au centre de son origine, déclenchant le premier mode de propagation électromagnétiqueTM01 dans le guide d'ondes à plaques parallèles. Une partie de l'énergie de l'onde est perdue par les fentes et une partie se propage. Dans le cas où toute l'énergie n'est pas évacuée par les fentes, l'onde progressive est réfléchie par le bord court-circuité au moyen de ladite au moins une rangée 24 de via(s) métallique(s) créant une réflexion arrière et par conséquent une onde stationnaire. L'onde rétro-réfléchie frappe aussi la fente et fuit par la fente.

Autrement dit, selon le mode de réalisation de la figure 2, ledit au moins un élément rayonnant planaire est un élément rayonnant à ondes fuyantes correspondant à au moins un patch à fente(s) annulaire(s), la troisième couche 16 comprenant, de part et d'autre dudit au moins un patch à fente(s) annulaire(s) $19_A$, au moins une rangée de via(s) métallique(s) 24 transversale s'étendant verticalement du plan de masse métallique au plan de la quatrième couche 18 comprenant ledit au moins un patch à fente(s) annulaire(s), ladite au moins une ligne d'alimentation 17 s'étendant verticalement du centre de la première couche d'alimentation 12 de la figure 1 au centre dudit au moins un patch à fente(s) annulaire(s) $19_A$.

Ainsi, selon la présente invention, pour une sommation constructive des deux ondes de fuite, la position du court-circuit via chaque rangée 24 de via(s) métallique(s) notamment matérialisée par la distance 26, et la position des fentes de l'élément rayonnant à ondes fuyantes du dispositif antennaire 10 sont au préalable définies de sorte que le dispositif antennaire 10 soit bien configuré pour superposer une pluralité de modes électromagnétiques en champ proche. Une telle définition préalable est notamment obtenue par optimisation de la sommation (i.e. superposition) des modes électromagnétiques rayonnés en champ proche au moyen de simulations notamment via l'outil de simulation tridimensionnel HFSS®.

La figure 3 illustre une vue en coupe d'une modélisation via l'outil de simulation tridimensionnel HFSS® d'un dispositif antennaire 10 selon la présente invention, un tel dispositif antennaire 10 comprenant à une antenne cylindrique à ondes de fuite illuminant la peau.

Comme détaillé précédemment en relation avec la figure 1, un tel modèle de simulation HFSS du dispositif antennaire 10 selon la présente invention comprend l'empilement de couches 12, 14, 16, 18, 20 et 22 précédemment décrites.

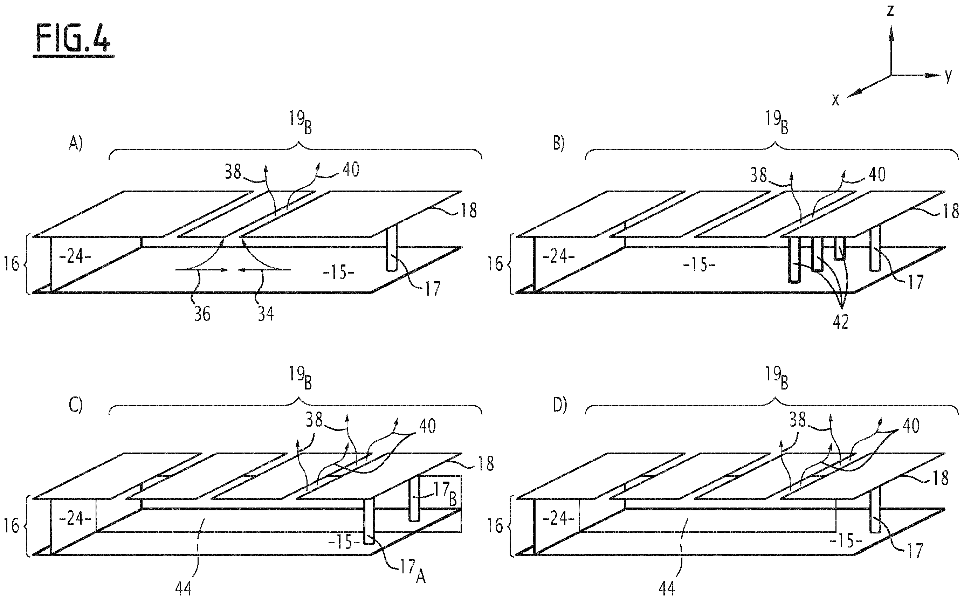

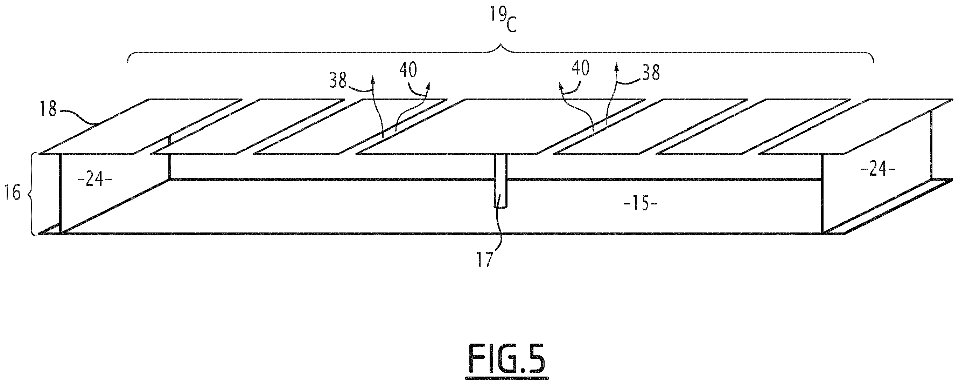

Le guide d'ondes à plaques parallèles, correspondant aux métallisations du plan de masse métallique 15 et au métallisations de l'élément rayonnant planaire, correspondant sur la figure 3 à un patch à fente(s) annulaire(s) $19_A$, ou à un patch à tronçons transversaux continus $19_B$ tel qu'illustré par la suite par les figures 4 et 5, est alimenté directement par la sortie radiofréquence RF de l'ASIC 28, localisé sous le substrat de la couche 12, via la broche coaxiale d'alimentation 17. La couche diélectrique 20 entre la peau et l'antenne est nécessaire pour permettre la superposition des modes cylindriques en champ proche. Des courts-circuits sont réalisés par des vias métalliques 30, en particulier, les vias métalliques 30 de la couche 16 correspondent à ladite au moins une rangée 24 de via(s) métallique(s) transversale définie précédemment en relation avec la figure 2, et les vias métalliques 30 de la couche 12 correspondent à des courts-circuits permettant de lier les deux couches d'alimentation 12 et 14.

Les deux couches du circuit d'alimentation en courant continu DC correspondent à des métallisations 32 localisées verticalement de part et d'autre du substrat de la couche 12 nécessaires à l'alimentation en courant continu DC et des plots de couplage 33 (de l'anglais *coupling stub*) pour assurer une continuité de masse et compenser la présence d'un film de liaison préimprégné 14 propre à produire une discontinuité de la masse, une telle continuité de masse permettant d'optimiser la transition entre l'ASIC 28 de la première couche 12 et la ligne d'alimentation 17, dont la base est équivalente à un coaxial.

Comme illustré par la figure 3, l'antenne à modes cylindriques en tant que telle ne nécessite que deux niveaux métalliques $19_A$ (ou $19_B$) pour la ou les fente(s) rayonnante(s) et 15 pour le plan de masse séparant, la couche d'antenne 16, des couches métalliques d'alimentation de la couche 12 comprenant l'ASIC 28 et deux couches de niveaux métalliques 32 inférieur et supérieur utilisés pour les circuits d'alimentation en courant continu.

Par exemple, une surface de rayonnement de 10mm x 10mm est adaptée à l'utilisation d'un patch à trois fentes annulaires $19_A$.

La figure 4 illustre différentes variantes A), B), C), D) de l'invention où l'élément rayonnant à ondes fuyantes correspond à un patch à tronçons transversaux continus $19_B$.

En particulier selon la variante A), le patch à tronçons transversaux continus $19_B$ du plan 18 repose sur la troisième couche 16 comprenant, localisée sensiblement à proximité d'une première extrémité transversale de ladite troisième couche 16, au moins une rangée de via(s) métallique(s) transversale 24 (i.e. selon la direction x) s'étendant verticalement (selon la direction z) du plan de masse métallique 15 au plan de la quatrième couche 18 comprenant ledit au moins un patch à tronçons transversaux continus $19_B$, ladite au moins une ligne d'alimentation 17, s'étendant verticalement de la première couche 12 illustrée par les figures 1 et 3 à la quatrième couche 18 en étant sensiblement localisée au niveau d'une deuxième extrémité transversale, de la troisième couche 16, parallèle et opposée à la première extrémité transversale de ladite troisième couche 16 localisée sensiblement à proximité de ladite au moins une rangée de via(s) métallique(s) 24 transversale, ladite au moins une ligne d'alimentation 17 étant propre à exciter le premier mode transverse magnétique TM01 dudit au moins un patch à tronçons transversaux continus $19_B$.

Autrement dit, le dispositif antennaire 10 correspond selon la variante A à un réseau de fentes cylindriques court-circuité sur l'un de ces bords transversaux (selon la direction x) par au moins une rangée de via(s) métallique(s) 24 transversale.

Plus précisément, le mode TM01, lancé au sein de l'antenne (formée des couches 16 et 18), excite selon les flèches 34 chaque fente à l'intérieur de l'antenne avec une amplitude et une phase données. Ce mode TM01 excité par la broche d'alimentation 17 crée une onde cylindrique qui fuit sur chaque fente. Cette onde est réfléchie selon les flèche 36 par la paroi court-circuitée sur le bord du guide d'onde à plaque parallèle opposé à ladite alimentation 17, et crée une onde réfléchie. Ainsi sur chaque fente sont présentes deux ondes excitées et réfléchies créant respectivement des modes rayonnants dominant 38 et réfléchis 40. La somme de ces deux modes définit les modes cylindriques superposés en champ proche et ainsi concentrés dans la zone réactive de traitement de la peau par le dispositif antennaire 10 selon cette variante A.

Les variantes B), C) et D) de la figures 4 visent à exploiter les propriétés de la superposition de N premiers modes dominants en utilisant également l'onde progressive ou stationnaire comme alimentation en série des fentes et visent des applications plus compactes que celle associée à la variante A).

La prise en compte des N premiers modes dans la sommation (i.e. superposition) de modes électromagnétiques obtenue au moyen du dispositif antennaire 10 selon la présente invention, permet de simplifier la conception de l'antenne (formée des couches 16 et 18). En effet, dans l'hypothèse d'une sommation de type monomode, il n'y a aucune relation avec le rayon de la fente et toute la conception dépend de la position et de la taille des fentes (équivalentes à la phase et à l'amplitude).

La variante B), est similaire à la variante A) précédemment décrite à la différence près que le patch à tronçons transversaux continus $19_B$ comprend trois fente au lieu de deux selon la variante A) et que la troisième couche 16 comprend en outre, entre ladite au moins une rangée de via(s) métallique(s) transversale 24 et ladite au moins une ligne d'alimentation 17, au moins deux autres vias métalliques 42 s'étendant verticalement du plan de masse métallique 15 au plan de la quatrième couche 18 comprenant ledit au moins un patch à tronçons transversaux continus $19_B$.

Autrement dit, pour une application plus compacte que celle visée selon la variante A), la variante B) introduit un deuxième mur métallique transversal formé desdits au moins deux autres vias métalliques 42.

De plus, par rapport à la variante de la figure 5 décrite ci-après, l'utilisation des vias métalliques 42 permet en outre d'appliquer une transformation du front d'onde cylindrique en front d'onde plan, et d'obtenir une uniformisation de la phase et des champs rayonnants.

L'utilisation des vias métallisés 42 est propre à limiter la possibilité de formation d'une onde plane à une fréquence plus élevée que celle désirée et les variantes C) et D) basées sur l'utilisation de deux sections de guide d'ondes sont proposées selon la présente invention pour y remédier.

En d'autres termes, dans les deux variantes C) et D), ledit au moins un élément rayonnant planaire est un élément rayonnant à ondes fuyantes correspondant à au moins un patch à tronçons transversaux continus $19_B$, la troisième couche 16 comprenant, localisé sensiblement à proximité d'une première extrémité transversale de ladite troisième couche 16, au moins une rangée de via(s) métallique(s) 24 transversale (selon la direction x) s'étendant verticalement (selon la direction z) du plan de masse métallique 15 au plan de la quatrième couche 18 comprenant ledit au moins un patch à tronçons transversaux continus $19_B$, et comprenant au moins deux guides d'onde longitudinaux (selon la direction y de la figure 4) obtenus par insertion au sein de la troisième couche 16 d'au moins une autre rangée de via(s) métallique(s) longitudinale 44 perpendiculaire, selon une direction longitudinale y de la figure 4, à la fois à ladite au moins une rangée de via(s) métallique(s) transversale 24 (selon la direction x) et au plan de masse métallique 15, ladite au moins une autre rangée de via(s) métallique(s) longitudinale 44 s'étendant verticalement (selon la direction z) du plan de masse métallique 15 au plan de la quatrième couche 18 comprenant ledit au moins un patch à tronçons transversaux continus $19_B$.

Selon la variante C), ladite au moins une rangée de via(s)métallique(s) longitudinale 44 s'étend longitudinalement, selon y, à partir de ladite au moins une rangée de via(s) métallique(s) transversale 24, et au-delà de la deuxième extrémité transversale de la troisième couche 16, parallèle et opposée à la première extrémité transversale de ladite troisième couche 16 localisée sensiblement à proximité de ladite au moins une rangée de via(s)métallique(s) transversale 24, le dispositif antennaire 10 comprenant alors au moins deux lignes d'alimentation $17_A$ et $17_B$, s'étendant verticalement de la première couche 12 à la quatrième couche 18 en étant localisées transversalement de part et d'autre de ladite au moins une rangée de via(s)

Selon la variante D), ladite au moins une rangée de via(s)métallique(s) longitudinale 44 s'étend longitudinalement, selon y, à partir de ladite au moins une rangée de via(s) métallique(s) transversale 24 et au-delà de la dernière fente dudit au moins un patch à tronçons transversaux continus $19_B$ tout en s'arrêtant à distance d'une deuxième extrémité transversale, de la troisième couche, parallèle et opposée à la première extrémité transversale de ladite troisième couche localisée sensiblement à proximité de ladite au moins une rangée de via(s) métallique(s) transversale 24, ladite au moins une ligne d'alimentation 17, s'étendant verticalement, selon z, de la première couche 12 à la quatrième couche 18 en étant localisée longitudinalement, selon y, entre :

- l'extrémité de ladite au moins une rangée de via(s) métallique(s) longitudinale 44 opposée à ladite au moins une rangée de via(s) métallique(s) transversale 24, et
- la deuxième extrémité transversale, de la troisième couche, parallèle et opposée à la première extrémité transversale de ladite troisième couche localisée sensiblement à proximité de ladite au moins une rangée de via(s) métallique(s) transversale 24.

Autrement dit, les figures variantes C) et D) montrent des fentes longues alimentées par deux sections de guide d'ondes excitées respectivement par deux et une seule broche 17.

La figure 5 montre une variante alternative de la figure 2 où à la place d'un patch à fente annulaires $19_A$, ledit au moins un élément rayonnant planaire est un élément rayonnant à ondes fuyantes correspondant à au moins un patch à tronçons transversaux continus $19_B$, la troisième couche 16 comprenant, de part et d'autre dudit au moins un patch à tronçons transversaux continus, au moins une rangée de via(s) métallique(s) transversale s'étendant verticalement du plan de masse métallique au plan de la quatrième couche comprenant ledit au moins un patch à tronçons transversaux continus, ladite au moins une ligne d'alimentation s'étendant verticalement du centre de la première couche au centre de la quatrième couche comprenant ledit au moins un patch à tronçons transversaux continus, les fentes dudit au moins un patch à tronçons transversaux continus étant réparties symétriquement de part et d'autre du point d'alimentation fourni par la ligne d'alimentation 17 au sein de la quatrième couche.

Cette alternative de la figure 5 présente ainsi des courts-circuits 24 sur les bords transversaux pour permettre son utilisation dans des applications compactes. De plus, une alimentation simple par broche 17 est appliquée. Dans la structure proposée de la figure 5, la propagation du mode TM01 excité dans le guide d'ondes à plaques parallèles se propage dans toutes les directions. Des ondes de fuite avec une différence de phase de 180° lorsque les fentes sont symétriques par rapport au point d'alimentation de la ligne d'alimentation 17 sont alors obtenues, de même qu'un mode dominant 38 et un mode réfléchi 40 dans la zone réactive du fait de la présence des courts-circuits 24.

Du fait de la paroi de court-circuitage 24 sur l'un des bords, le mode TM01 dominant et réfléchi rayonne dans la zone réactive mais présente un front d'onde cylindrique, qui crée un retard de phase sur les fentes et un rayonnement non uniforme.

Pour y remédier, les variantes B), C) et D) précédemment décrites, et tel que notamment vérifié par simulation, permettent en outre d'appliquer une transformation du front d'onde cylindrique en front d'onde plan par rapport à la variante de la figure 5, d'obtenir une uniformisation de la phase et des champs rayonnants superposés ce qui crée une distribution de puissance quasi-uniforme sur la peau, et de fournir un éclairage efficace de la peau avec une consommation d'énergie presque deux fois plus faible.

Selon un aspect non représenté, la combinaison (i.e. superposition) des modes est en outre propre à être réalisée électroniquement afin de réaliser une formation de faisceau électronique dans la région de champ proche. Par exemple, cette combinaison peut être implémentée par des éléments parasites charges avec des composants réglables tels que des capacités, résistances et/ou inductances.

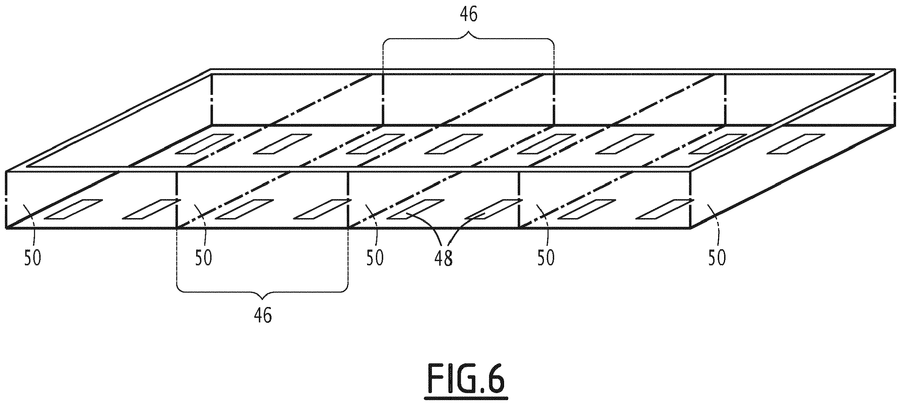

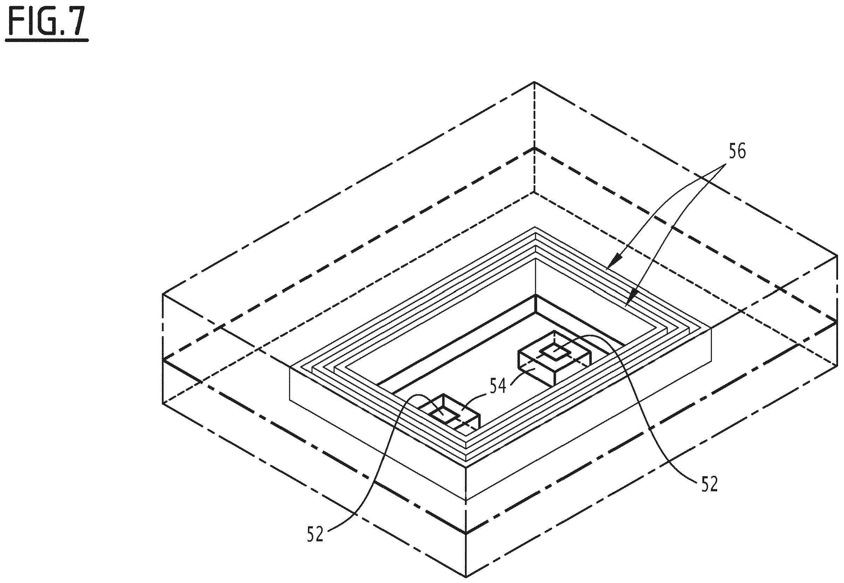

Les figures 6 et 7 illustrent différentes variantes d'un mode de réalisation de l'architecture proposée dans lequel les modes électromagnétiques propres à être superposés selon l'invention comprennent en outre des modes cavité (i.e. rectangulaires) propres à être excités par ajout d'au moins une cavité métallique au sein de la structure multicouche selon la présente invention

Dans les solutions/variantes proposées précédemment en relation avec les figures 1 à 5 pour une illumination efficace de la peau pour la thérapie par ondes millimétriques, la superposition de N modes cylindriques a été proposée, et selon le mode de réalisation complémentaire et optionnel des figures 6 et 7, il est également proposé, selon la présente invention, d'utiliser d'autres types de modes tels que des modes cavités par utilisation d'au moins une cavité pour des modes d'ordre supérieur. Selon le mode, une excitation différente doit être imposée.

La figure 6 représente la couche 16 d'un réseau d'antennes composé de quatre cavités 46 (de taille supérieure à A la longueur d'onde). L'excitation des cavités 46 formées par quatre murs métalliques est réalisée par N fentes 48 qui permettent de créer un mode de cavité d'ordre supérieur. L'antenne (formée des couches 16 et 18 précitées), chargée par le couvercle 20 de séparation avec la peau 22, crée une distribution de puissance strictement liée à l'ordre du mode cavité créé. En configurant préalablement par simulation les modes de cavité pour chaque ouverture de cavité 46, il est possible de réduire le couplage entre les ouvertures de cavité 46 et augmenter ainsi l'efficacité du dispositif proposé selon la présente invention.

Les variantes illustrées par les figures 1 à 6 présentent différents dispositifs antennaires, présentant l'empilement de la figure 1, et illuminant la peau 22 avec une couverture diélectrique séparatrice 20. De manière connue, la densité de puissance dans la peau 22 est exponentielle atténuée, ce qui rend essentiel de concentrer tous les composants du champ dans la pénétration.

Les ondes de fuite excitées selon les variantes 1 à 6 présentent une dissipation de puissance dans tous les axes, à savoir en x, y et z. La composante normale à la face de la peau 22, le long de l'axe z, contribue à la pénétration dans la peau tandis que les autres composantes peuvent être traitées comme une perte de fuite.

Les champs qui pénètrent directement dans la peau (énergie de la composante z du vecteur de Poynting) peuvent être de l'ordre de la moitié de la densité de puissance totale excitée par la source. Les autres composantes du champ s'échappent progressivement vers la peau ou s'échappent par les bords de la couverture en plastique 20 comprenant le radôme.

Pour y remédier, la figure 7 illustre une extension des dispositifs antennaires précédemment proposés, avec un élément rayonnant planaire 52 correspondant à un patch (ou aux éléments rayonnant planaires présentés précédemment $19_A$ et $19_B$) entouré d'une cavité 54, et basée sur la superposition des modes en tant que milieu semi-fini délimité par des parois métalliques 56. En effet, le milieu à l'intérieur des parois métalliques 56 est propre à créer une cavité de taille supérieure à deux longueurs d'onde guidées et supporte ainsi des modes d'ordre supérieur. Ces parois métalliques 56 peuvent notamment être introduites dans la cinquième couche de couverture 20 en créant une cavité supplémentaire et en minimisant les effets de fuite.

Une telle cavité ouverte formée des parois métalliques 56 est propre à conduire à une désadaptation et à des fuites par la petite fente entre la cavité et la peau. Pour éviter ces effets, il est proposé de terminer les parois métalliques 56 de la cavité par des plots (i.e. stubs) d'adaptation

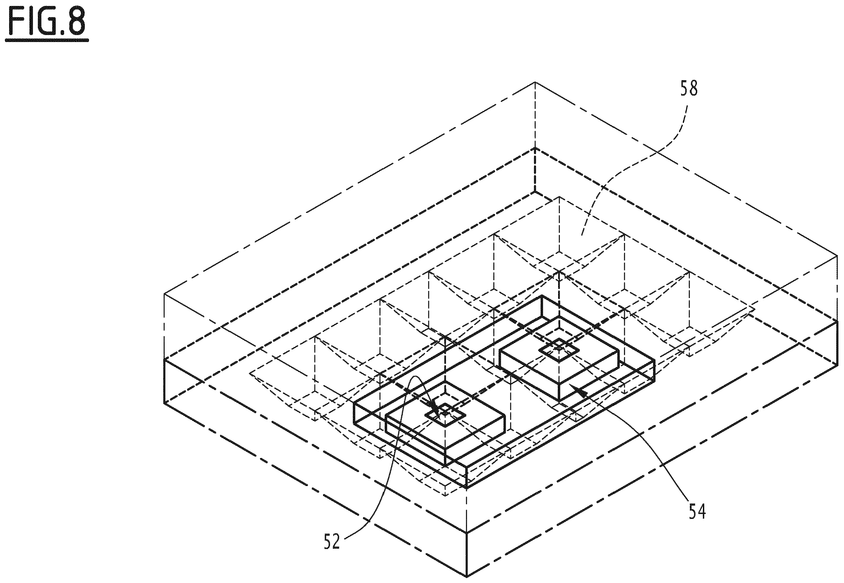

En complément optionnel, tel qu'illustré par la figure 8, le radôme de la cinquième couche de couverture 20 est configuré pour être une couverture diélectrique modulée 58 afin de réduire les fuites de puissance par le côté de la couverture 20.

Plus précisément, il est connu d'utiliser un diélectrique modulé mis à la terre pour transformer des ondes de surface en ondes de fuite ou pour optimiser un rayonnement en champ lointain. Selon la présente invention, il est spécifiquement proposé d'utiliser des phénomènes similaires dans la zone réactive entre le dispositif antennaire et la peau pour capturer les ondes de surface créées dans la couverture diélectrique et les transformer en illumination de la peau.

La figure 8 montre un réseau de deux éléments rayonnants planaire 52 au sein d'une cavité 54 chargés par un diélectrique modulé 58.

Pour une conception classique d'une telle surface modulée 58 pour un dispositif antennaire à ondes de fuite, le rayon de l'élément rayonnant doit être de plusieurs longueurs d'onde, alors que pour une application compacte un autre type de modulation en fonction du type d'élément rayonnant planaire utilisé est appliqué tel qu'un motif régulier en forme de pyramide pour des éléments rayonnants correspondant à des antennes de type patch.

Une telle couverture diélectrique modulée est, selon la présente invention, propre à être considérée comme une couche d'adaptation qui permet une meilleure concentration des champs dans la zone réactive, et une réduction de la dissipation de puissance s'échappant par les bords de la couverture, est obtenue, ce qui permet d'améliorer la réduction des fuites.

L'homme du métier comprendra que l'invention ne se limite pas aux modes de réalisation décrits, ni aux exemples particuliers de la description, les modes de réalisation et les variantes mentionnées ci-dessus étant propres à être combinés entre eux pour générer de nouveaux modes de réalisation de l'invention.

La présente invention propose ainsi une solution pour l'illumination uniforme et ponctuelle en champ proche de la peau. Ce dispositif antennaire est propre à être utilisé pour l'application de la thérapie médicale par ondes millimétriques, pour l'acupuncture et la stimulation des endorphines à 1-100 GHz, pour la détection épidermique du cancer, ou encore pour augmenter la capacité de pénétration des ondes électromagnétiques dans les tissus humains ou plus généralement dans les milieux inhomogènes.

La nouvelle architecture de dispositif antennaire est simple à mettre en œuvre grâce à la séparation entres les couches supérieures 16, 18 et 20 RF et les couches métalliques inférieures de la couche 12 permettant une réduction du couplage mutuel entre ces couches RF et DC, une réduction de perte et par conséquent de consommation d'énergie.

De plus, l'utilisation, selon la présente invention, de la transition entre onde(s) stationnaire(s) et onde de fuite(s) ne nécessite pas de diviseur de puissance à pertes.

Enfin, la superposition de modes générée au moyen du dispositif antennaire 10 selon la présente invention permet de créer efficacement un champ proche avec une distribution quasi-uniforme ou multiple des points chauds.

La superposition de modes ainsi que la transition entre onde(s) stationnaire(s) et onde de fuite(s) utilisées selon la présente invention n'ont pas été utilisées jusqu'ici pour la thérapie par ondes millimétriques afin d'obtenir une distribution uniforme du champ proche et des multiples points chauds.

**Revendications**

1. Dispositif antennaire (10) d'illumination en champ proche de la peau (22) par ondes millimétriques **caractérisé en ce que** ledit dispositif (10) est configuré pour superposer une pluralité de modes électromagnétiques en champ proche, en utilisant une structure multicouche comprenant, selon une direction verticale (z) d'empilement :

   - une première couche (12) d'alimentation comprenant au moins un premier diélectrique sur un circuit intégré pour application spécifique (28) et sur une première partie inférieure d'un circuit (32) métallique d'alimentation en courant continu,
   - une deuxième couche d'alimentation en courant continu, superposée sur la première couche d'alimentation selon la direction verticale d'empilement (z), et comprenant une deuxième partie supérieure d'un circuit (32) métallique d'alimentation en courant continu,
   - une troisième couche (16), superposée sur la deuxième couche d'alimentation selon la direction verticale d'empilement (z), et comprenant un deuxième diélectrique sur un plan métallique (15),
   - une quatrième couche (18) comprenant au moins un élément rayonnant planaire ($19_A$, $19_B$) dudit dispositif antennaire, la quatrième couche (18) étant superposée sur la troisième couche (16) selon la direction verticale d'empilement (z), le plan métallique (15) de la troisième couche (16) jouant le rôle de plan de masse de l'élément rayonnant ($19_A$, $19_B$),
   - une cinquième couche (20), superposée sur la quatrième couche (18) selon la direction verticale d'empilement (z), et comprenant un radôme propre à séparer ledit au moins un élément rayonnant ($19_A$, $19_B$) de la peau (22),

   le dispositif antennaire (10) comprenant en outre au moins une ligne d'alimentation (17) s'étendant verticalement de la première couche (12) à la quatrième couche (18).

2. Dispositif antennaire (10) selon la revendication 1, dans lequel les modes électromagnétiques propres à être superposés sont des modes cylindriques.

3. Dispositif antennaire (10) selon la revendication 1 ou 2, dans lequel ledit au moins un élément rayonnant planaire est un élément rayonnant à ondes fuyantes correspondant à au moins un patch à fente(s) annulaire(s) ($19_A$), ou à au moins un patch à tronçons transversaux continus ($19_B$).

4. Dispositif antennaire (10) selon l'une quelconque des revendications précédentes dans lequel la troisième couche (16) comprend au moins une rangée (24) de via(s) métallique(s) transversale définie par la direction verticale d'empilement (z) et une direction transversale (x) perpendiculaire à la direction verticale d'empilement (z), ladite au moins une rangée (24) de via(s) métallique(s) transversale s'étendant verticalement du plan de masse métallique (15) de la troisième couche (16) au plan de la quatrième couche (18) comprenant ledit au moins un élément rayonnant planaire ($19_A$, $19_B$).

5. Dispositif antennaire (10) selon la revendication 4, dans lequel ledit au moins un élément rayonnant planaire est un élément rayonnant à ondes fuyantes correspondant à au moins un patch à fente(s) annulaire(s) ($19_A$), la troisième couche (16) comprenant, de part et d'autre dudit au moins un patch à fente(s) annulaire(s) ($19_A$,), au moins une rangée (24) de via(s) métallique(s) transversale s'étendant verticalement du plan de masse métallique (15) au plan de la quatrième couche (18) comprenant ledit au moins un patch à fente(s) annulaire(s) ($19_A$), ladite au moins une ligne d'alimentation (17) s'étendant verticalement du centre de la première couche (12) d'alimentation au centre dudit au moins un patch à fente(s) annulaire(s) ($19_A$).

6. Dispositif antennaire (10) selon la revendication 4, dans lequel ledit au moins un élément rayonnant planaire est un élément rayonnant à ondes fuyantes correspondant à au moins un patch à tronçons transversaux continus ($19_B$), la troisième couche (16) comprenant, de part et d'autre dudit au moins un patch à tronçons transversaux continus, au moins une rangée (24) de via(s) métallique(s) transversale s'étendant verticalement du plan de masse métallique (15) au plan de la quatrième couche (18) comprenant ledit au moins un patch à tronçons transversaux continus ($19_B$), ladite au moins une ligne d'alimentation (17) s'étendant verticalement du centre de la première couche (12) au centre de la quatrième couche (18) comprenant ledit au moins un patch à tronçons transversaux continus ($19_B$), les fentes dudit au moins un patch à tronçons transversaux continus ($19_B$) étant réparties symétriquement de part et d'autre du point d'alimentation fourni par la ligne d'alimentation au sein de la quatrième couche (18).

7. Dispositif antennaire (10) selon la revendication 4, dans lequel ledit au moins un élément rayonnant planaire est un élément rayonnant à ondes fuyantes correspondant à au moins un patch à tronçons transversaux continus ($19_B$), la troisième couche (16) comprenant, localisée sensiblement à proximité d'une première extrémité transversale de ladite troisième couche (16), au moins une rangée (24) de via(s) métallique(s) transversale s'étendant verticalement du plan de masse métallique (15) au plan de la quatrième couche (18) comprenant ledit au moins un patch à tronçons transversaux continus ($19_B$), ladite au moins une ligne d'alimentation (17), s'étendant verticalement de la première couche (12) à la quatrième couche (18) en étant sensiblement localisée au niveau d'une deuxième extrémité transversale, de la troisième couche (16), parallèle et opposée à la première extrémité transversale de ladite troisième couche (16) localisée sensiblement à proximité de ladite au moins une rangée (24) de via(s) métallique(s) transversale, ladite au moins une ligne d'alimentation (17) étant propre à exciter le premier mode transverse magnétique dudit au moins un patch à tronçons transversaux continus ($19_B$).

8. Dispositif antennaire (10) selon la revendication la revendication 7, comprenant en outre entre ladite au moins une rangée (24) de via(s) métallique(s) transversale et ladite au moins une ligne d'alimentation (17) au moins deux autres vias métalliques (42) s'étendant verticalement du plan de masse métallique (15) au plan de la quatrième couche (18) comprenant ledit au moins un patch à tronçons transversaux continus.

9. Dispositif antennaire (10) selon la revendication 4, dans lequel ledit au moins un élément rayonnant planaire est un élément rayonnant à ondes fuyantes correspondant à au moins un patch à tronçons transversaux continus ($19_B$), la troisième couche (16) comprenant, localisé sensiblement à proximité d'une première extrémité transversale de ladite troisième couche (16), au moins une rangée (24) de via(s) métallique(s) transversale s'étendant verticalement du plan de masse métallique (15) au plan de la quatrième couche (18) comprenant ledit au moins un patch à tronçons transversaux continus ($19_B$), et comprenant au moins deux guides d'onde longitudinaux obtenus par insertion au sein de la troisième couche (16) d'au moins une autre rangée (44) de via(s) métallique(s) longitudinale perpendiculaire, selon une direction longitudinale (y), à la fois à ladite au moins une rangée (24) de via(s) métallique(s) transversale et au plan de masse métallique (15), ladite au moins une autre rangée (44) de via(s) métallique(s) longitudinale s'étendant verticalement du plan de masse métallique (15) au plan de la quatrième couche (18) comprenant ledit au moins un patch à tronçons transversaux continus ($19_B$).

10. Dispositif antennaire (10) selon la revendication la revendication 9, dans lequel ladite au moins une rangée (44) de via(s)métallique(s) longitudinale s'étend longitudinalement à partir de ladite au moins une rangée (24) de via(s) métallique(s) transversale et au-delà de la dernière fente dudit au moins un patch à tronçons transversaux continus tout en s'arrêtant à distance d'une deuxième extrémité transversale, de la troisième couche, parallèle et opposée à la première extrémité transversale de ladite troisième couche (16) localisée sensiblement à proximité de ladite au moins une rangée (24) de via(s) métallique(s) transversale, ladite au moins une ligne d'alimentation (17), s'étendant verticalement de la première couche (12) à la quatrième couche (18) en étant localisée longitudinalement entre :

- l'extrémité de ladite au moins une rangée (44) de via(s) métallique(s) longitudinale opposée à ladite au moins une rangée de via(s) métallique(s) transversale, et
- la deuxième extrémité transversale, de la troisième couche (16), parallèle et opposée à la première extrémité transversale de ladite troisième couche (16) localisée sensiblement à proximité de ladite au moins une rangée (24) de via(s) métallique(s) transversale.

11. Dispositif antennaire (10) selon la revendication la revendication 9, dans lequel ladite au moins une rangée (44) de via(s)métallique(s) longitudinale s'étend longitudinalement à partir de ladite au moins une rangée (24) de via(s) métallique(s) transversale et au-delà de la deuxième extrémité transversale, de la troisième couche (16), parallèle et opposée à la première extrémité transversale de ladite troisième couche localisée sensiblement à proximité de ladite au moins une rangée (24) de via(s)métallique(s) transversale,
le dispositif antennaire comprenant alors au moins deux lignes d'alimentation ($17_A$, $17_B$), s'étendant verticalement de la première couche (12) à la quatrième couche (18) en étant localisées transversalement de part et d'autre de ladite au moins une rangée (44) de via(s)métallique(s) longitudinale.

12. Dispositif antennaire (10) selon l'une quelconque des revendications précédentes 2 à 11, dans lequel les modes électromagnétiques propres à être superposés comprennent en outre des modes cavité propres à être excités par ajout d'au moins une cavité métallique (46, 54) au sein de la structure multicouche.

13. Dispositif antennaire (10) selon l'une quelconque des revendications précédentes, dans lequel le radôme (20) est configuré pour être une couverture diélectrique modulée (58).

## FIG.1

**FIG.2**

## FIG.3

FIG.4

FIG.5

EP 4 102 729 A1

**FIG.6**

FIG.7

FIG.8

**Europäisches Patentamt**
**European Patent Office**
**Office européen des brevets**

# RAPPORT DE RECHERCHE EUROPEENNE

Numéro de la demande

**EP 22 17 7556**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 6 184 828 B1 (SHOKI HIROKI [JP]) 6 février 2001 (2001-02-06) | 1-8,12, 13 | INV. H04B5/00 |
| A | * colonne 36, alinéas 2,4,6 * * colonne 34, alinéa 5 * * figures 1,3,25,49,52, * * colonne 35, alinéa 1-4 * * colonne 11, alinéa dernier – colonne 12, alinéa 1 * * colonne 9, alinéas 4, dernier – colonne 10, alinéa 1 * * colonne 2, dernier alinéa * * colonne 19, alinéas 2,4 * ----- | 9-11 | |

**DOMAINES TECHNIQUES RECHERCHES (IPC)**

H04B
H01Q

Le présent rapport a été établi pour toutes les revendications

| Lieu de la recherche | Date d'achèvement de la recherche | Examinateur |
|---|---|---|
| Munich | 17 juin 2022 | Panahandeh, Ali |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04C02)

22

## ANNEXE AU RAPPORT DE RECHERCHE EUROPEENNE
## RELATIF A LA DEMANDE DE BREVET EUROPEEN NO.

EP 22 17 7556

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

17-06-2022

| Document brevet cité au rapport de recherche | Date de publication | Membre(s) de la famille de brevet(s) | Date de publication |
|---|---|---|---|
| US 6184828 B1 | 06-02-2001 | JP H06326510 A | 25-11-1994 |
| | | US 6184828 B1 | 06-02-2001 |

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 3071163 A1

**Littérature non-brevet citée dans la description**

- **M. SMIERZCHALSKI.** Characterization methods for metamaterials. Directive antennas using space eigenmodes. Univ. Rennes, 2014, vol. 1